(19) **Europäisches Patentamt European Patent Office Office européen des brevets**

(11) **EP 3 984 598 B1**

(12) **EUROPEAN PATENT SPECIFICATION**

(45) Date of publication and mention of the grant of the patent:
**18.10.2023 Bulletin 2023/42**

(51) International Patent Classification (IPC):
**A61P 25/00** *(2006.01)*　**A61P 25/16** *(2006.01)*
**A61K 9/70** *(2006.01)*　**A61K 47/06** *(2006.01)*
**A61K 47/10** *(2017.01)*　**A61K 47/32** *(2006.01)*
**A61K 31/381** *(2006.01)*

(21) Application number: **20822737.1**

(22) Date of filing: **05.06.2020**

(52) Cooperative Patent Classification (CPC):
**A61K 9/7053; A61K 31/381; A61K 47/10; A61P 25/00; A61P 25/16**

(86) International application number:
**PCT/JP2020/022410**

(87) International publication number:
**WO 2020/250840 (17.12.2020 Gazette 2020/51)**

(54) **ROTIGOTINE-CONTAINING PATCH**

ROTIGOTINHALTIGES PFLASTER

TIMBRE CONTENANT DE LA ROTIGOTINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.06.2019 JP 2019111313**

(43) Date of publication of application:
**20.04.2022 Bulletin 2022/16**

(73) Proprietor: **Hisamitsu Pharmaceutical Co., Inc.**
**Tosu-shi, Saga 841-0017 (JP)**

(72) Inventors:
• **ARAKI, Hiroyuki**
**Tsukuba-shi, Ibaraki 305-0856 (JP)**
• **TAKAGI, Yuka**
**Tsukuba-shi, Ibaraki 305-0856 (JP)**

• **FUJIWARA, Yoko**
**Tsukuba-shi, Ibaraki 305-0856 (JP)**
• **KOBAYASHI, Hiroaki**
**Tsukuba-shi, Ibaraki 305-0856 (JP)**
• **KUROKAWA, Takao**
**Tsukuba-shi, Ibaraki 305-0856 (JP)**

(74) Representative: **Grünecker Patent- und Rechtsanwälte**
**PartG mbB**
**Leopoldstraße 4**
**80802 München (DE)**

(56) References cited:
**WO-A1-2006/082888　WO-A1-2012/084969**
**WO-A1-2019/124261　WO-A1-2019/142940**
**JP-A- 2013 079 220　JP-B2- 5 753 645**

**Description**

[Technical Field]

The present invention relates to a rotigotine-containing patch, and more particularly to a patch containing rotigotine and/or a pharmaceutically acceptable salt thereof.

[Background Art]

Rotigotine is an international general name of a compound (-)-5,6,7,8-tetrahydro-6- [propyl- [2- (2-thienyl)ethyl]-amino] 1-naphthalenol. Rotigotine is a D1/D2/D3 dopamine receptor agonist, and is mainly used for the treatment of symptoms of Parkinson's disease and restless legs syndrome.

For example, as a formulation for rotigotine administration, "Neupro (registered trademark) Patch" is commercially available in Japan and overseas. In addition, Japanese Unexamined Patent Application Publication No. 2010-159302 (PTL 1) describes a transdermal therapeutic system comprising a self-adhesive matrix layer containing rotigotine as a trans-epicutaneous administration form for treating restless legs syndrome, and International Application Japanese-Phase Publication No. 2002-509878 (PTL 2) describes a transdermal therapeutic system comprising a backing layer inert to the components of a matrix, and a self-adhesive matrix layer containing rotigotine, wherein the matrix contains, as a base, a non-aqueous, acrylate-based or silicone-based polymer adhesive having a solubility of 5% (w/w) for rotigotine. Furthermore, International Publication No. WO2012/084969 (PTL 3) describes a transdermal absorption system comprising an adhesive agent layer formed of an adhesive agent composition containing styrene-based polymer and/or polyisobutylene, rotigotine or a pharmaceutically acceptable salt thereof, and polyvinylpyrrolidone or vinylpyrrolidone and/or vinyl acetate.

In addition, for example, Japanese Unexamined Patent Application Publication No. 2014-177428 (PTL 4) describes a transdermal absorption-type patch formulation comprising a support, and a drug-containing layer which includes a rubber-based adhesive agent containing a rosin-based resin and a rubber-based adhesive component, and which includes rotigotine or a pharmaceutically acceptable salt thereof, and Japanese Unexamined Patent Application Publication No. 2018-12331 (PTL 5) describes a transdermal absorption-type patch comprising a support, a drug-containing layer, and a release liner, wherein the drug-containing layer contains rotigotine, a polyisobutylene-based adhesive agent, liquid paraffin, and polyvinylpyrrolidone. JP2013079220 relates to a transdermal patch comprising a support, a drug-containing layer containing rotigotine , and an inhibitor of the formation of a degradation product of rotigotine.

[Citation List]

[Patent Literature]

[PTL 1] Japanese Unexamined Patent Application Publication No. 2010-159302
[PTL 2] International Application Japanese-Phase Publication No. 2002-509878
[PTL 3] International Publication No. WO2012/084969
[PTL 4] Japanese Unexamined Patent Application Publication No. 2014-177428
[PTL 5] Japanese Unexamined Patent Application Publication No. 2018-123131 [PTL 6] Japanese Patent Application Publication No. JP2013079220

[Summary of Invention]

[Technical Problem]

However, as a result of further examination on a rotigotine-containing patch containing rotigotine and/or a pharmaceutically acceptable salt thereof, the present inventors found the following problem. Specifically, although a large number of agents have been conventionally known as tackifiers and drug transdermal absorption promoters to be contained in patches, the present inventors have found that even containing the above agents, conventional rotigotine-containing patches containing rotigotine and/or a pharmaceutically acceptable salt thereof may still fail to achieve sufficient skin permeability of rotigotine.

The present invention has been made in view of the above problem, and an object thereof is to provide a patch with excellent skin permeability of rotigotine.

[Solution to Problem]

The present inventors have made earnest studies to achieve the above object, and have consequently found that a patch including a backing layer and an adhesive agent layer, when the adhesive agent layer contains at least one selected from the group consisting of rotigotine and pharmaceutically acceptable salts thereof (hereinafter referred to as "rotigotine and/or a pharmaceutically acceptable salt thereof" in some cases) in combination with an alicyclic saturated hydrocarbon resin and a specific aliphatic alcohol, achieves a particularly higher level of skin permeability of rotigotine than in the case of using other tackifiers or transdermal absorption promoters. Thus, the present invention has been completed.

Specifically, a rotigotine-containing patch of the present invention is a rotigotine-containing patch comprising:

a backing layer; and
an adhesive agent layer, wherein
the adhesive agent layer contains rotigotine and/or a pharmaceutically acceptable salt thereof, and
the adhesive agent layer further contains an alicyclic saturated hydrocarbon resin and at least one aliphatic alcohol selected from the group consisting of lauryl alcohol, octyldodecanol, oleyl alcohol, and myristyl alcohol.

In the rotigotine-containing patch of the present invention, it is preferable that a content of the aliphatic alcohol in the adhesive agent layer is 1 to 15% by mass relative to a total mass of the adhesive agent layer. It is also preferable that a content of rotigotine and/or a pharmaceutically acceptable salt thereof in the adhesive agent layer in terms of rotigotine free form is 5 to 15% by mass relative to the total mass of the adhesive agent layer.

Furthermore, in the rotigotine-containing patch of the present invention, it is preferable that a content of the alicyclic saturated hydrocarbon resin in the adhesive agent layer is 5 to 80% by mass relative to the total mass of the adhesive agent layer, and it is also preferable that the adhesive agent layer further contains a styrene-based thermoplastic elastomer. It is more preferable that a content of the styrene-based thermoplastic elastomer in the adhesive agent layer is 5 to 50% by mass relative to the total mass of the adhesive agent layer.

Furthermore, in the rotigotine-containing patch of the present invention, it is preferable that the adhesive agent layer further contains a plasticizer, and it is more preferable that a content of the plasticizer in the adhesive agent layer is 4 to 50% by mass relative to the total mass of the adhesive agent layer.

[Advantageous Effects of Invention]

According to the present invention, it is possible to provide a rotigotine-containing patch with particularly excellent skin permeability of rotigotine.

[Description of Embodiments]

The invention is described in detail with reference to suitable embodiments thereof. A rotigotine-containing patch of the present invention is a rotigotine-containing patch comprising:

a backing layer; and
an adhesive agent layer, wherein
the adhesive agent layer contains rotigotine and/or a pharmaceutically acceptable salt thereof, and
the adhesive agent layer further contains an alicyclic saturated hydrocarbon resin and at least one aliphatic alcohol selected from the group consisting of lauryl alcohol, octyldodecanol, oleyl alcohol, and myristyl alcohol.

The rotigotine-containing patch of the present invention includes a backing layer and an adhesive agent layer. The backing layer is not particularly limited as long as it can support the adhesive agent layer to be described later, and a known backing layer for a patch can be appropriately employed. Examples of the material of the backing layer according to the present invention include polyolefins such as polyethylene and polypropylene; ethylene-vinyl acetate copolymer, vinyl acetate-vinyl chloride copolymer, polyvinyl chloride, and the like; polyamides such as nylon; polyesters such as polyethylene terephthalate (PET), polybutylene terephthalate, and polyethylene naphthalate; cellulose derivatives; and synthetic resins such as polyurethane, and metals such as aluminum. Among these, polyesters and polyethylene terephthalate are preferable from the viewpoint of non-adsorbability and non-permeability of drugs. Examples of the form of the backing layer include films; sheet-shaped objects such as sheets, sheet-shaped porous bodies, and sheet-shaped foams; fabrics such as woven fabrics, knitted fabrics, and nonwoven fabrics; foils; and laminates thereof. In addition, the thickness of the backing layer is not particularly limited, but is preferably in the range of 5 to 1000 $\mu$m from the viewpoints of workability and ease of production when applying the patch.

The rotigotine-containing patch of the present invention may further include a release liner on the surface of the adhesive agent layer opposite to the backing layer. Examples of such release liner include polyolefins such as polyethylene and polypropylene; ethylene-vinyl acetate copolymer, vinyl acetate-vinyl chloride copolymer, polyvinyl chloride, and the Like; polyamides such as nylon; polyesters such as polyethylene terephthalate; cellulose derivatives; and films and sheets made of materials such as synthetic resins including polyurethane, aluminum, and paper, and laminates thereof. Preferably, these release liners have been subjected to a release treatment using a silicone-containing compound coat, a fluorine-containing compound coat, or the like on the surface in contact with the adhesive agent layer so as to easily enable release from the adhesive agent layer.

<Rotigotine and Pharmaceutically Acceptable Salt Thereof >

The adhesive agent layer according to the present invention contains at least one selected from the group consisting of rotigotine and pharmaceutically acceptable salts thereof as a drug. In the present invention, the form of rotigotine contained in the adhesive agent layer may be a free form or a pharmaceutically acceptable salt thereof, may be a pharmaceutically acceptable salt of rotigotine that has been desalted into a free form in the formulation during production and/or after production, or may be one of these or a mixture of two or more thereof. Examples of the pharmaceutically acceptable salt of rotigotine include acid addition salts, and examples of the acid addition salts include hydrochloric acid, sulfuric acid, nitric acid, phosphoric acid, phosphorous acid, hydrobromic acid, maleic acid, malic acid, ascorbic acid, tartaric acid, lauric acid, stearic acid, palmitic acid, oleic acid, myristic acid, lauryl sulfate, linolenic acid, and fumaric acid. Among these, the adhesive agent layer according to the present invention preferably contains rotigotine in a free form.

In the present invention, the content of rotigotine and/or a pharmaceutically acceptable salt thereof contained in the adhesive agent layer (the content of rotigotine, the content of a pharmaceutically acceptable salt of rotigotine, or the total content thereof if both of them are contained, hereinafter the same) is, in terms of rotigotine free form, preferably 5 to 15% by mass, more preferably 7 to 14% by mass, further preferably 7 to 12% by mass, still further preferably 8 to 12% by mass, and particularly preferably 8 to 10% by mass relative to the total mass of the adhesive agent layer. When the content of rotigotine and/or a pharmaceutically acceptable salt thereof is less than the lower limit, the skin permeability of rotigotine tends to decrease. Meanwhile, when the upper limit is exceeded, there is a tendency that crystals of rotigotine and/or a pharmaceutically acceptable salt thereof are precipitated, an amorphous type is formed, and the adhesive force of the adhesive agent layer is easily reduced.

<Alicyclic Saturated Hydrocarbon Resin>

The adhesive agent layer of the present invention further contains an alicyclic saturated hydrocarbon resin. In the present invention, a particularly high level of skin permeability is achieved when the adhesive agent layer contains the alicyclic saturated hydrocarbon resin in combination with the following specific aliphatic alcohol. It is known that the alicyclic saturated hydrocarbon resin mainly functions as a tackifier for adhesive base agents, but if other tackifiers are contained instead of the alicyclic saturated hydrocarbon resin in the adhesive agent layer according to the present invention, the skin permeability of rotigotine tends to decrease.

The alicyclic saturated hydrocarbon resin according to the present invention refers to an alicyclic hydrogenated petroleum resin that is a homopolymer or copolymer of alicyclic saturated hydrocarbon monomers. The alicyclic saturated hydrocarbon resin has a weight average molecular weight of preferably 1,000 to 1,500, and more preferably 1,200 to 1,400. As the alicyclic saturated hydrocarbon resin according to the present invention, one of these may be used alone, or two or more may be used in combination. More specific examples of the alicyclic saturated hydrocarbon resin include Arkon P-70, Arkon P-85, Arkon P-90, Arkon P-100, Arkon P-115, Arkon P-125 (these are trade names, manufactured by Arakawa Chemical Industries, Ltd.).

In the present invention, the content of the alicyclic saturated hydrocarbon resin contained in the adhesive agent layer (if the alicyclic saturated hydrocarbon resin is a combination of two or more kinds, the total content thereof, hereinafter the same) is preferably 5 to 80% by mass, more preferably 10 to 70% by mass, further preferably 10 to 60% by mass, still further preferably 20 to 60% by mass, and particularly preferably 20 to 50% by mass relative to the total mass of the adhesive agent layer. When the content of the alicyclic saturated hydrocarbon resin is less than the lower limit, the adhesive force of the adhesive agent layer and the adhesion to the skin tend to decrease. Meanwhile, when the upper limit is exceeded, the transdermal absorbability of rotigotine and the shape retainability of the adhesive agent layer tend to decrease. From the viewpoint of the transdermal absorbability of rotigotine, it is also preferable that the content of the alicyclic saturated hydrocarbon resin is 10 to 50% by mass.

<Aliphatic Alcohol>

The adhesive agent layer of the present invention further contains at least one aliphatic alcohol selected from the group consisting of lauryl alcohol, octyldodecanol, oleyl alcohol, and myristyl alcohol. Note that in the present invention, the aliphatic alcohol refers to a saturated or unsaturated, linear or branched, monohydric or dihydric or higher aliphatic alcohol. In the rotigotine-containing patch, sufficient skin permeability of rotigotine tends not to be exhibited even when an aliphatic alcohol other than these four types is used, and furthermore, when an aliphatic alcohol having 6 or less carbon atoms is used, the boiling point is lowered, so that it is difficult to keep the content in the formulation constant, and the stability over time tends to decrease.

In the present invention, the content of the aliphatic alcohol contained in the adhesive agent layer (if the aliphatic alcohol is a combination of two or more kinds, the total content thereof, hereinafter the same) is preferably 1 to 15% by mass, more preferably 1 to 10% by mass, further preferably 2 to 10% by mass, still further preferably 2 to 7% by mass, and particularly preferably 3 to 7% by mass in total relative to the total mass of the adhesive agent layer. When the content of the aliphatic alcohol is less than the lower limit, the skin permeability of rotigotine tends to decrease. Meanwhile, when the upper limit is exceeded, the compatibility with the adhesive base agent and other components tends to decrease.

In addition, in the present invention, the mass ratio of the content of the alicyclic saturated hydrocarbon resin to the content of the aliphatic alcohol contained in the adhesive agent layer (content of the alicyclic saturated hydrocarbon resin:content of the aliphatic alcohol) is preferably 17:1 to 2:1, more preferably 12:1 to 3:1, and further preferably 10:1 to 5:1. When the content of the alicyclic saturated hydrocarbon resin to the content of the aliphatic alcohol is less than the lower limit, the adhesive force of the adhesive agent layer and the adhesion to the skin tend to decrease. Meanwhile, when upper limit is exceeded, the transdermal absorbability of rotigotine tends to decrease. From the viewpoint of transdermal absorbability of rotigotine, it is also preferable that the mass ratio is 10:1 to 2:1.

<Adhesive Base Agent>

The adhesive agent layer according to the present invention contains an adhesive base agent. The adhesive base agent is not particularly limited, and examples thereof include rubber-based adhesive base agents, acrylic-based adhesive base agents, and silicone-based adhesive base agents, and one of these may be used alone, or two or more may be used in combination, but it is preferable to contain at Least a rubber-based adhesive base agent.

(Rubber-Based Adhesive Base Agent)

Examples of the rubber-based adhesive base agents include styrene-based thermoplastic elastomers, isoprene rubber, polyisobutylene (PIB), and polybutene, and one of these may be used alone, or two or more may be used in combination, and among these, the styrene-based thermoplastic elastomer is particularly preferable. The styrene-based thermoplastic elastomer is a thermoplasticity-exhibiting styrene-based elastomer which exhibits fluidity by softening when heated, and which returns to a rubber-like elastic body when cooled. Among these, a styrene-based block copolymer is preferred from the viewpoint of sufficient tackiness impartment and stability over time.

Specific examples of the styrene-based block copolymer include styrene-butadiene block copolymer, styrene-butadiene-styrene block copolymer (SBS), styrene-isoprene block copolymer, styrene-isoprene-styrene block copolymer (SIS), styrene-ethylene/butylene block copolymer, styrene-ethylene/butylene-styrene block copolymer, styrene-ethylene/propylene block copolymer, styrene-ethylene/propylene-styrene block copolymer, styrene-isobutylene block copolymer, and styrene- isobutylene-styrene block copolymer, and one of these may be used alone, or two or more may be used in combination. Note that, in the above, "ethylene/butylene" indicates a copolymer block of ethylene and butylene, and "ethylene/propylene" indicates a copolymer block of ethylene and propylene. Among these, the styrene-based thermoplastic elastomer according to the present invention is more preferably a styrene-isoprene-styrene block copolymer.

The styrene-isoprene-styrene block copolymer has a viscosity average molecular weight of preferably 30,000 to 2,500,000, and more preferably 100,000 to 1,700,000. When the viscosity average molecular weight is less than the lower limit, the formulation properties of the patch (particularly the cohesive force of the adhesive agent layer) tend to decrease. Meanwhile, when the upper limit is exceeded, there is a tendency that the compatibility with other components contained in the adhesive agent layer is reduced, making it difficult to produce a patch.

In the present invention, when the styrene-based thermoplastic elastomer is contained in the adhesive agent layer as the adhesive base agent, the content thereof (if the styrene-based thermoplastic elastomer is a combination of two or more kinds, the total content thereof, hereinafter the same) is preferably 5 to 50% by mass, more preferably 10 to 40% by mass, and further preferably 10 to 30% by mass relative to the total mass of the adhesive agent layer. When the content of the styrene-based thermoplastic elastomer is less than the lower limit, the cohesive force, shape retainability, and the like of the adhesive agent layer tend to decrease. Meanwhile, when the upper limit is exceeded, there is a tendency that the cohesive force of the adhesive agent layer excessively increases, so that the adhesive force of the adhesive agent layer decreases or the compatibility decreases.

In addition, from the viewpoint that the tackiness and cohesive force of the adhesive agent layer tend to be further improved, the rubber-based adhesive base agent is preferably a combination of a styrene-based thermoplastic elastomer (more preferably a styrene-isoprene-styrene block copolymer) and polyisobutylene, and it is further preferable that the mass ratio of the styrene-based thermoplastic elastomer to polyisobutylene (mass of the styrene-based thermoplastic elastomer:mass of PIB) is 1:2 to 30:1 (further preferably in the range of 1:1 to 10:1).

In the present invention, when a rubber-based adhesive base agent is contained in the adhesive agent layer as the adhesive base agent, the content thereof (in the case of a combination of two or more kinds, the total content thereof, hereinafter the same) is preferably 1 to 60% by mass, more preferably 5 to 50% by mass, and further preferably 10 to 40% by mass relative to the total mass of the adhesive agent layer.

(Acrylic-Based Adhesive Base Agent)

Examples of the acrylic-based adhesive base agents are listed in "Japanese Pharmaceutical Excipients Directory 2016 (edited by International Pharmaceutical Excipients Council Japan)" as adhesive agents, such as acrylic acid-octyl acrylate ester copolymer, 2-ethylhexyl acrylate-vinyl pyrrolidone copolymer, acrylate ester-vinyl acetate copolymer, 2-ethylhexyl acrylate-2-ethylhexyl methacrylate-dodecyl methacrylate copolymer, methyl acrylate·2-ethylhexyl acrylate copolymer resin, 2-ethylhexyl acrylate-methyl acrylate-acrylic acid-glycidyl methacrylate copolymer, 2-ethylhexyl acrylate-vinyl acetate-hydroxyethyl acrylate·glycidyl methacrylate copolymer, 2-ethylhexyl acrylate-diacetone acrylamide·acetoacetoxyethyl methacrylate-methyl methacrylate copolymer, ethyl acrylate-methyl methacrylate copolymer, acrylic-based polymer contained in an alkanolamine solution of acrylic resin, and one of these may be used alone, or two or more may be used in combination.

(Silicone-Based Adhesive Base Agent)

Examples of the silicone-based adhesive base agents include polydimethylsiloxane (such as the polymer represented by MQ in the representation by ASTM D-1418), polymethylvinylsiloxane (such as the polymer represented by VMQ in the representation by ASTM D-1418), and polymethylphenylsiloxane (such as the polymer represented by PVMQ in the representation by ASTM D-1418), and one of these may be used alone, or two or more may be used in combination.

In the present invention, when the acrylic-based adhesive base agent and/or silicone-based adhesive base agent is contained in the adhesive agent layer as the adhesive base agent, the content thereof (in the case of a combination of two or more kinds, the total content thereof, hereinafter the same) is preferably 10% by mass or less relative to the total mass of the adhesive agent layer.

<Additional Components>

As long as the effects of the present invention are not impaired, the adhesive agent layer according to the present invention may further contain additives such as additional drugs other than rotigotine and a pharmaceutically acceptable salt thereof; additional tackifiers other than the alicyclic saturated hydrocarbon resin; additional absorption promoters other than the aliphatic alcohol; adsorbents, desalting agents, plasticizers, solubilizers, fillers, stabilizers, and preservatives.

(Additional Drugs)

Examples of the additional drugs other than rotigotine and the pharmaceutically acceptable salt thereof include nonsteroidal anti-inflammatory analgesics (such as diclofenac, indomethacin, ketoprofen, felbinac, loxoprofen, ibuprofen, flurbiprofen, tiaprofen, acemetacin, sulindac, etodolac, tolmetin, piroxicam, meloxicam, ampiroxicam, naproxen, azapropazone, methyl salicylate, glycol salicylate, valdecoxib, celecoxib, rofecoxib, and amfenac), antipyretic analgesics (such as acetaminophen), antihistamines (such as diphenhydramine, chlorpheniramine, mequitazine, and homochlorcyclizine), antihypertensives (such as diltiazem, nicardipine, nilvadipine, metoprolol, bisoprolol, and trandolapril), antiparkinsonian drugs (such as pergolide, ropinirole, bromocriptine, and selegiline), bronchodilators (such as tulobuterol, isoproterenol, and salbutamol), antiallergic agents (such as ketotifen, loratadine, azelastine, terfenadine, cetirizine, and acitazanolast), local anesthetics (such as lidocaine and dibucaine), neuropathic pain medications (such as pregabalin), non-narcotic analgesics (buprenorphine, tramadol, pentazocine), anesthetic analgesics (such as morphine, oxycodone, and fentanyl), agents for urinary organs (such as oxybutynin and tamsulosin), psychotropic agents (such as promazine and chlorpromazine), steroid hormones (such as estradiol, progesterone, norethisterone, cortisone, and hydrocortisone), antidepressants (such as sertraline, fluoxetine, paroxetine, and citalopram), anti-dementia drugs (such as donepezil, rivastigmine, and galantamine), antipsychotics (such as risperidone and olanzapine), central nervous system stimulants (such as methylphenidate), osteoporosis medications (such as raloxifene and alendronate), breast cancer prevention drugs (such as tamoxifen), anti-obesity drugs (such as mazindol and sibutramine), insomnia improving drugs (such as melatonin), and antirheumatic drugs (such as actarit), and one of these may be used alone, or two or more may be used in combination.

In the present invention, consider the case where these additional drugs are further contained in the adhesive agent layer. The content thereof is, in the case of two or more kinds, the total content thereof, preferably 10% by mass or less relative to the total mass of the adhesive agent layer.

(Additional Tackifier)

Examples of additional tackifiers other than the alicyclic saturated hydrocarbon resins include petroleum-based resins other than the alicyclic saturated hydrocarbon resins, terpene-based resins, rosin-based resins, phenol-based resins, and xylene-based resins, and one of these may be used alone , or two or more may be used in combination.

Examples of the petroleum-based resins other than the alicyclic saturated hydrocarbon resins include alicyclic hydrogenated petroleum resins, aliphatic petroleum resins (such as aliphatic hydrocarbon resins), aliphatic hydrogenated petroleum resins, and aromatic petroleum resins, and more specific examples include Escorez 8000 (trade name, manufactured by Esso). As the petroleum-based resins, one of these may be used alone, or two or more may be used in combination.

Examples of the terpene-based resin include pinene polymers (such as $\alpha$-pinene polymers and $\beta$-pinene polymers), terpene polymers, dipentene polymers, terpene-phenol polymers, aromatic modified terpene polymers, and pinene-phenol copolymers. More specific examples include YS RESIN (such as YS RESIN PXN (1150N, 300N), YS RESIN PX1000, YS RESIN TO125, and YS RESIN TO105), CLEARON P105, CLEARON M115, CLEARON K100 (these are trade names, manufactured by YASUHARA CHEMICAL CO., LTD.), and Tamanol 901 (trade name, manufactured by Arakawa Chemical Industries, Ltd.), and one of these may be used alone, or two or more may be used in combination.

Examples of the rosin-based resins include hydrogenated rosin glycerin esters, super light-colored rosin, super light-colored rosin esters, and acid-modified super light-colored rosin, and more specific examples include Pinecrystal (such as KE-311, PE-590, KE-359, and KE-100) (these are trade names, manufactured by Arakawa Chemical Industries, Ltd.), and one of these may be used alone, or two or more may be used in combination.

In the present invention, consider the case where these additional tackifiers are further contained in the adhesive agent layer. The content thereof is, in the case of two or more kinds, the total content thereof, preferably 10% by mass or less relative to the total mass of the adhesive agent layer.

(Additional Absorption Promoter (Transdermal Absorption Promoter))

Examples of the absorption promoters include those having an effect of promoting the transdermal absorption of drugs other than the specific aliphatic alcohol, such as aliphatic alcohols other than the specific aliphatic alcohol, fatty acids having 6 to 20 carbon atoms, fatty acid esters, fatty acid amides, or aliphatic alcohol ethers; aromatic organic acids; aromatic alcohols; aromatic organic acid esters or ethers; POE hydrogenated castor oils; lecithins; phospholipids; soybean oil derivatives; and triacetin, and one of these may be used alone, or two or more may be used in combination.

Examples of the aliphatic alcohol other than the specific aliphatic alcohol include isopropanol, hexyl alcohol, cetyl alcohol, stearyl alcohol, isostearyl alcohol, linolenyl alcohol, and hexyldecanol, and one of these may be used alone, or two or more may be used in combination.

Examples of absorption promoters other than the aliphatic alcohol include fatty acids having 6 to 20 carbon atoms, fatty acid esters, fatty acid amides, or aliphatic alcohol ethers; aromatic organic acids; aromatic alcohols; aromatic organic acid esters or ethers; POE hydrogenated castor oils; lecithins; phospholipids; soybean oil derivatives; and triacetin, and one of these may be used alone, or two or more may be used in combination.

In the present invention, consider the case where these additional absorption promoters are further contained in the adhesive agent layer. The content thereof is, in the case of two or more kinds, the total content thereof, preferably 10% by mass or less relative to the total mass of the adhesive agent layer.

(Additive)

[Adsorbent]

Examples of the adsorbents include hygroscopic inorganic and/or organic substances, and more specific examples thereof include minerals such as talc, kaolin, and bentonite; silicon compounds such as fumed silica (such as AEROSIL (registered trademark)) and hydrous silica; metal compounds such as zinc oxide and dried aluminum hydroxide gel; weak acids such as lactic acid and acetic acid; sugars such as dextrin; and polymers such as polyvinylpyrrolidone (non-cross-linked PVP), cross-linked polyvinylpyrrolidone (also referred to as "crospovidone" or "cross-linked PVP"), aminoalkyl methacrylate copolymer, carboxyvinyl polymer, and butyl methacrylate methyl methacrylate copolymer, and one of these may be used alone, or two or more may be used in combination.

Examples of the cross-linked polyvinylpyrrolidone include a cross-linked N-vinylpyrrolidone polymer. The N-vinylpyrrolidone polymer may be a homopolymer or a copolymer, and examples thereof include a homopolymer of N-vinylpyrrolidone and a copolymer of N-vinylpyrrolidone and a polyfunctional monomer. Among these, the cross-linked polyvinylpyrrolidone according to the present invention is preferably a cross-linked homopolymer of 1-vinyl-2-pyrrolidone. As the cross-linked polyvinylpyrrolidone, commercially available ones may be used, such as Kollidon CL and Kollidon CL-M (manufactured by BASF Japan) ; and Polyplasdone XL, Polyplasdone XL-10, and Polyplasdone IMF-10 (manufactured by ISP Japan).

In the present invention, consider the case where these adsorbents are further contained in the adhesive agent layer. The content thereof is, in the case of two or more kinds, the total content thereof, preferably 10% by mass or less relative to the total mass of the adhesive agent layer.

In addition, in the present invention, when the adhesive agent layer contains the cross-linked polyvinylpyrrolidone (preferably, the mass ratio to the content of the rotigotine and/or a pharmaceutically acceptable salt thereof (rotigotine free form equivalent content of rotigotine and/or a pharmaceutically acceptable salt thereof:cross-linked polyvinylpyrrolidone content) is 10:3 to 1:3), it is possible to suppress the precipitation of crystals of rotigotine and/or a pharmaceutically acceptable salt thereof, and from the viewpoint of the particularly excellent skin permeability of rotigotine, it is preferable that the cross-linked polyvinylpyrrolidone is not contained in the adhesive agent layer, and the content thereof is preferably 10% by mass or less, and more preferably 8% by mass or less (for example, 3 to 8% by mass), relative to the total mass of the adhesive agent layer.

[Desalting Agent]

The desalting agent is blended mainly for the purpose of converting all or a part of the basic drug into a free form. Such a desalting agent is not particularly limited, but is preferably, for example, a basic substance, and more preferably a metal ion-containing desalting agent or a basic nitrogen atom-containing desalting agent in the case of blending an acid addition salt of drug as the drug to obtain a formulation containing a free form drug. Examples of the metal ion-containing desalting agent include sodium acetate (including anhydrous sodium acetate), sodium hydroxide, potassium hydroxide, magnesium hydroxide, sodium hydrogen carbonate, potassium hydrogen carbonate, sodium citrate, and sodium lactate, and one of these may be used alone, or two or more may be used in combination. Note that the adhesive agent layer according to the present invention may further contain a compound derived from the basic drug and the desalting agent (for example, when rotigotine hydrochloride and sodium acetate are combined, sodium hydrochloride). In the present invention, consider the case where these desalting agents and compounds derived from basic drugs and desalting agents are further contained in the adhesive agent layer. The content thereof is, in the case of two or more kinds, the total content thereof, preferably 10% by mass or less relative to the total mass of the adhesive agent layer.

[Plasticizer]

The plasticizer is blended mainly for the purpose of adjusting the adhesive properties of the adhesive agent layer, flow characteristics in the production of the adhesive agent layer, transdermal absorption characteristics of the drug, and the like. Examples of such a plasticizer include silicone oils; petroleum-based oils such as paraffinic process oils, naphthenic process oils, and aromatic process oils; squalane, squalene; vegetable-based oils such as olive oil, camellia oil, castor oil, tall oil, and peanut oil; dibasic acid esters such as dibutyl phthalate and dioctyl phthalate; liquid rubbers such as polybutene and liquid isoprene rubber; and diethylene glycol, polyethylene glycol, propylene glycol, and dipropylene glycol, and one of these may be used alone, or two or more may be used in combination. Among these, at least one selected from the group consisting of silicone oil, liquid paraffin, and liquid polybutene is preferable as the plasticizer. In the present invention, consider the case where plasticizers are further contained in the adhesive agent layer. The content thereof is, in the case of two or more kinds, the total content thereof, preferably 4 to 50% by mass, more preferably 5 to 30% by mass, further preferably 10 to 20% by mass, and still further preferably 15 to 20% by mass relative to the total mass of the adhesive agent layer, from the viewpoint of improving the adhesive force of the adhesive agent layer and/or alleviating local irritation during release. From the viewpoint of transdermal absorbability of rotigotine, the content of the plasticizer is also preferably 15 to 50% by mass.

[Solubilizer·Filler]

Examples of the solubilizers include organic acids such as acetic acid, and surfactants, and one of these may be used alone, or two or more may be used in combination. In addition, the filler is blended mainly for the purpose of adjusting the adhesive force of the adhesive agent layer, and examples of the filler include aluminum hydroxide, calcium carbonate, and magnesium carbonate; silicates such as aluminum silicate and magnesium silicate; and silicic acid, barium sulfate, calcium sulfate, calcium zincate, zinc oxide, and titanium oxide, and one of these may be used alone, or two or more may be used in combination.

[Stabilizer]

Examples of the stabilizers include ascorbic acid, metal salts, or esters thereof (preferably sodium salts and palmitate esters), isoascorbic acid or metal salts thereof (preferably sodium salts), ethylenediaminetetraacetic acid or metal salts thereof (preferably calcium disodium salts and tetrasodium salts), cysteine, acetylcysteine, 2-mercaptobenzimidazole, dibutylhydroxytoluene, butylhydroxyanisole, propyl gallate, pentaerythrityl-tetrakis[3-(3,5-di-t-butyl-4-hydroxyphenyl)propionate], 3-mercapto-1,2-propanediol, tocopherol acetate, thymol, soy lecithin, rutin, dihydroxybenzoic acid, potassium dichloroisocyanurate, quercetin, hydroquinone, metal salts of hydroxymethanesulfinic acid (preferably sodium salts), metal metabisulfites (such as sodium salts), metal sulfites (preferably sodium salts), and metal thiosulfates (preferably sodium salt), and one of these may be used alone, or two or more may be used in combination. In the above, examples of the metal salts include sodium salts, potassium salts, calcium salts, and magnesium salts. In addition, examples of the esters include palmitate esters, stearate esters, and myristate esters.

In the present invention, when the adhesive agent layer further contains the stabilizer, the stability over time tends to further improve, and from the viewpoint of the particularly excellent skin permeability of rotigotine, it is preferable that the stabilizer (such as 2-mercaptobenzimidazole) is not contained in the adhesive agent layer, and the content thereof is preferably 3% by mass or less, more preferably 2% by mass or less, and further preferably 0.25% by mass or less (for example, 0.005 to 0.25% by mass) relative to the total mass of the adhesive agent layer.

[Preservative]

Examples of the preservatives include derivatives of paraoxybenzoic acid, benzyl alcohol, phenol, and cresol, and one of these may be used alone, or two or more may be used in combination.

Consider the case where the above additives are further contained in the adhesive agent layer. The content thereof is, in the case of two or more kinds, the total content thereof, preferably 40% by mass or less, and more preferably 30% by mass or less relative to the total mass of the adhesive agent layer.

The adhesive agent layer according to the present invention is not particularly limited, but has a mass per unit area (area of the sticking surface) of preferably 20 to 200 $g/m^2$, more preferably 30 to 100 $g/m^2$, and further preferably 30 to 70 $g/m^2$. In addition, the area of the sticking surface of the adhesive agent layer according to the present invention cari be appropriately adjusted depending on the purpose of treatment and the target of application, and is not particularly limited, but is usually in the range of 0.5 to 200 $cm^2$.

The rotigotine-containing patch of the present invention is not particularly limited, and can be produced by appropriately employing a known patch production method. For example, first, rotigotine and/or a pharmaceutically acceptable salt thereof, the alicyclic saturated hydrocarbon resin, the specific aliphatic alcohol, the adhesive base agent, and optionally a solvent and the additional components are kneaded in a usual manner to obtain a uniform adhesive agent layer composition. In the case of using a rotigotine free form as the rotigotine and/or the pharmaceutically acceptable salt thereof, the I-type crystals, II-type crystals, or amorphous form thereof may be used, or a mixture of at least two or more of the I-type crystals, II-type crystals, and amorphous form may be used. In addition, as the rotigotine and/or the pharmaceutically acceptable salt thereof, those dissolved in the solvent may be used. Examples of the solvent include anhydrous ethanol, toluene, heptane, methanol, ethyl acetate, hexane, and a mixture of at least two or more of these.

Next, this adhesive agent layer composition is applied onto the surface (usually onto one surface) of the backing layer to a desired mass per unit area, and then the solvent is dried and removed by heating as necessary to form an adhesive agent layer, which is further cut into a desired shape as necessary. Thereby, it is possible to obtain the patch of the present invention.

In addition, the method for producing a rotigotine-containing patch of the present invention may further include a step of attaching the release liner to the surface of the adhesive agent layer opposite to the backing layer, the step including first applying the adhesive agent layer composition onto one surface of the release liner to a desired mass per unit area to form an adhesive agent layer, then attaching the backing layer to the surface of the adhesive agent layer opposite to the release liner, and cutting the unit into a desired shape as necessary, thereby obtaining the patch of the present invention. Moreover, the obtained patch may be enclosed in a preservation packaging container (such as an aluminum laminate bag) as necessary to form a package.

[Examples]

Hereinafter, the present invention is described more specifically based on Examples and Comparative Examples, but the present invention is not limited to the following Examples. Note that, in each of Examples and Comparative Examples, the skin permeation test was performed by the following method.

<Skin Penetration Test (in vitro Hairless Mouse Skin Penetration Test)>

First, to the stratum corneum side of a fat-removed skin piece obtained by peeling the skin of the hairless mouse body to remove fat, a patch cut into a square of 1.0 cm$^2$ with the release liner removed was attached. In this way, a test sample was prepared. This was set in a flow-through type diffusion cell such that the dermis side was in contact with receptor solution, and the cell was filled with a receptor solution (phosphate buffered saline). Next, the receptor solution was sent at a flow rate of about 5 mL/hr while circulating warm circulating water around the outer periphery so that the receptor solution was kept at 32°C, and the receptor solution was collected every two hours up to 24 hours. The rotigotine concentration in the collected receptor solution was measured by high performance liquid chromatography, and the following formula:

$$\texttt{amount of skin permeation of rotigotine (}\mu\texttt{g/cm}^2\texttt{)}$$

$$\texttt{= \{rotigotine concentration in receptor solution}$$

$$\texttt{(}\mu\texttt{g/mL) × flow rate (mL)\}/area of patch (cm}^2\texttt{)}$$

was used calculate the rate of skin permeation of rotigotine per unit area of the adhesive agent layer, thereby obtaining the rate of skin permeation per hour (rate of skin permeation ($\mu$g/cm$^2$/hr)). Each measurement was performed on two test samples, and the average of the maximum values of rate of skin permeation within 24 hours was defined as the maximum rate of skin permeation (Jmax).

(Example 1)

First, to an appropriate amount of solvent (absolute ethanol and toluene), 9.0 parts by mass of rotigotine (free form), 15.4 parts by mass of styrene-isoprene-styrene block copolymer, 6.6 parts by mass of polyisobutylene, 48.4 parts by mass of alicyclic saturated hydrocarbon resin (Arkon P-100, manufactured by Arakawa Chemical Industries, Ltd.), 17.6 parts by mass of liquid paraffin, and 3 parts by mass of lauryl alcohol were added and mixed to obtain an adhesive agent layer composition. Next, the obtained adhesive agent layer composition was applied on a release liner (polyethylene terephthalate film subjected to release treatment), and the solvent was removed by drying to a mass per unit area of 50 g/m$^2$, thereby forming an adhesive agent layer. A backing layer (polyethylene terephthalate film) was stacked on the surface of the obtained adhesive agent layer opposite to the release liner to obtain a patch formed of a stack of backing layer/adhesive agent layer/release liner in this order.

(Examples 2 to 4 and Comparative Examples 1 to 18)

Each patch was obtained in the same manner as in Example 1 except that the composition of the adhesive agent layer composition was changed to the compositions shown in Table 1 or 2 below.
The patches obtained in Examples 1 to 4 and Comparative Examples 1 to 18 were subjected to a skin permeation test. Tables 1 and 2 show the results together with the compositions (excluding the solvent) of the adhesive agent layer compositions of Examples and Comparative Examples.

[Table 1]

| | Comp. Ex. 1 | Example 1 | Example 2 | Example 3 | Example 4 | Comp. Ex. 2 | Comp. Ex.3 | Comp. Ex.4 | Comp. Ex. 5 | Comp. Ex. 6 |
|---|---|---|---|---|---|---|---|---|---|---|
| Adhesive Agent Layer Composition [Parts by Mass] | | | | | | | | | | |
| Rotigotine | 9 | 9 | 9 | 9 | 9 | 9 | 9 | 9 | 9 | 9 |
| Styrene-Isoprene-Styrene Block Copolymer | 15.9 | 15.4 | 15.4 | 15.4 | 15.4 | 15.4 | 15.4 | 15.4 | 15.4 | 15.4 |
| Polyisobutylene | 6.8 | 6.6 | 6.6 | 6.6 | 6.6 | 6.6 | 6.6 | 6.6 | 6.6 | 6.6 |
| Alicyclic Saturated Hydrocarbon Resin | 50.1 | 48.4 | 48.4 | 48.4 | 48.4 | 48.4 | 48.4 | 48.4 | 48.4 | 48.4 |
| Liquid Paraffin | 18.2 | 17.6 | 17.6 | 17.6 | 17.6 | 17.6 | 17.6 | 17.6 | 17.6 | 17.6 |
| Lauryl Alcohol (C12) | - | 3 | - | - | - | - | - | - | - | - |
| Octyldodecanol (C20) | - | - | 3 | - | - | - | - | - | - | - |
| Oleyl Alcohol (C18) | - | - | - | 3 | - | - | - | - | - | - |
| Myristyl Alcohol (C14) | - | - | - | - | 3 | - | - | - | - | - |
| Isostearyl Alcohol (C18) | - | - | - | - | - | 3 | - | - | - | - |
| Stearyl Alcohol (C18) | - | - | - | - | - | - | 3 | - | - | - |
| Cetyl Alcohol (C16) | - | - | - | - | - | - | - | 3 | - | - |
| Isopropanol (C3) | - | - | - | - | - | - | - | - | 3 | - |
| Hexyl Alcohol (C6) | - | - | - | - | - | - | - | - | - | 3 |
| Total | 100 | 100 | 100 | 100 | 100 | 100 | 100 | 100 | 100 | 100 |
| Evaluation | | | | | | | | | | |
| Maximum Rate of Skin Permeation (Jmax) [$\mu$g/cm$^2$/hr] | 11.7 | 18.6 | 17.6 | 15.1 | 14.2 | 12.9 | 12.7 | 12.3 | 13.6 | 13.2 |
| Comp. Ex. : Comparative Example | | | | | | | | | | |

[Table 2]

| | Comp. Ex. 7 | Comp. Ex. 8 | Comp. Ex. 9 | Comp. Ex. 10 | Comp. Ex. 11 | Comp. Ex. 12 | Comp. Ex.13 | Comp. Ex.14 | Comp. Ex.15 | Comp. Ex. 16 | Comp. Ex.17 | Comp. Ex.18 |
|---|---|---|---|---|---|---|---|---|---|---|---|---|
| Adhesive Agent Layer Composition [Parts by Mass] | | | | | | | | | | | | |
| Rotigotine | 9 | 9 | 9 | 9 | 9 | 9 | 9 | 9 | 9 | 9 | 9 | 9 |
| Styrene-Isoprene-Styrene Block Copolymer | 15.4 | 15.4 | 15.4 | 15.4 | 15.4 | 15.4 | 15.4 | 15.4 | 15.4 | 15.4 | 15.4 | 15.4 |
| Polyisobutylene | 6.6 | 6.6 | 6.6 | 6.6 | 6.6 | 6.6 | 6.6 | 6.6 | 6.6 | 6.6 | 6.6 | 6.6 |
| Alicyclic Saturated Hydrocarbon Resin | 48.4 | 48.4 | 48.4 | 48.4 | 48.4 | 48.4 | 48.4 | 48.4 | 48.4 | 48.4 | 48.4 | 48.4 |
| Liquid Paraffin | 17.6 | 17.6 | 17.6 | 17.6 | 17.6 | 17.6 | 17.6 | 17.6 | 17.6 | 17.6 | 17.6 | 17.6 |
| Glycerol Monooleate | 3 | - | - | - | - | - | - | - | - | - | - | - |
| Alginic Acid | - | 3 | - | - | - | - | - | - | - | - | - | - |
| Betaine Lauryl Dimethylamino Acetate | - | - | 3 | - | - | - | - | - | - | - | - | - |
| Sorbitan Laurate | - | - | - | 3 | - | - | - | - | - | - | - | - |
| Dimethyl Sulfoxide | - | - | - | - | 3 | - | - | - | - | - | - | - |
| Diethanolamine | - | - | - | - | - | 3 | - | - | - | - | - | |
| Palmitic Acid | - | - | - | - | - | - | 3 | - | - | - | - | |
| Isopropanol Amine | - | - | - | - | - | - | - | 3 | - | - | - | |
| Triisopropanolamine | - | - | - | - | - | - | - | - | 3 | - | - | |
| Diisopropanolamine | - | - | - | - | - | - | - | - | - | 3 | - | |
| Sorbic Acid | - | - | - | - | - | - | - | - | - | - | 3 | - |
| Lactic Acid | - | - | - | - | - | - | - | - | - | - | - | 3 |
| Total | 100 | 100 | 100 | 100 | 100 | 100 | 100 | 100 | 100 | 100 | 100 | 100 |
| Evaluation | | | | | | | | | | | | |

EP 3 984 598 B1

12

| Evaluation | | | | | | | | | | | | |
|---|---|---|---|---|---|---|---|---|---|---|---|---|
| Maximum Rate of Skin Permeation (Jmax) [$\mu$g/cm$^2$/hr] | 11.8 | 11.4 | 11.0 | 11.0 | 10.8 | 10.1 | 7.1 | 6.8 | 5.7 | 5.7 | 5.7 | 4.5 |
| Comp. Ex. : Comparative Example | | | | | | | | | | | | |

EP 3 984 598 B1

As shown in Tables 1 and 2, excellent skin permeability of rotigotine was achieved in the patch of the present invention using lauryl alcohol, octyldodecanol, oleyl alcohol, or myristyl alcohol (Examples 1 to 4), and in particular, it was confirmed that particularly excellent skin permeability was achieved when compared to the case of using other aliphatic alcohols that were close in the number of carbon atoms or had the same number of carbon atoms as these (such as Comparative Examples 2 to 4) .

(Example 5 and Comparative Examples 19 and 20)

Each patch was obtained in the same manner as in Example 1 except that the composition of the adhesive agent layer composition was changed to the compositions shown in Table 3 below. In Table 3, the terpene-based resin used was "YS RESIN PX1150N (manufactured by YASUHARA CHEMICAL CO., LTD.)," and the hydrogenated rosin ester used was "KE-311 (manufactured by Arakawa Chemical Industries, Ltd.) ."

The patches obtained in Example 5 and Comparative Examples 19 and 20 were subjected to a skin permeation test. Table 3 shows the results together with the compositions (excluding the solvent) of the adhesive agent layer compositions of Example and Comparative Examples.

[Table 3]

| | Example 5 | Comparative Example 19 | Comparative Example 20 |
|---|---|---|---|
| Adhesive Agent Layer Composition [Parts by Mass] | | | |
| Rotigotine | 9 | 9 | 9 |
| Styrene-Isoprene-Styrene Block Copolymer | 13.3 | 13.3 | 13.3 |
| Polyisobutylene | 5.7 | 5.7 | 5.7 |
| Alicyclic Saturated Hydrocarbon Resin | 41.5 | - | - |
| Terpene-Based Resin | - | 41.5 | - |
| Hydrogenated Rosin Ester | - | - | 41.5 |
| Liquid Paraffin | 15.2 | 15.2 | 15.2 |
| Octyldodecanol | 5 | 5 | 5 |
| Cross-Linked PVP | 10 | 10 | 10 |
| Stabilizer (2-Mercaptobenzimidazole) | 0.3 | 0.3 | 0.3 |
| Total | 100 | 100 | 100 |
| Evaluation | | | |
| Maximum Rate of Skin Permeation (Jmax) [$\mu$g/cm$^2$/hr] | 15.8 | 13.6 | 6.7 |

As shown in Table 3, excellent skin permeability of rotigotine was achieved in the patch of the present invention using octyldodecanol and alicyclic saturated hydrocarbon resin (Example 5), and it was confirmed that sufficient skin permeability was achieved even when adsorbents and stabilizers were further contained. On the other hand, when other tackifiers such as terpene resins or hydrogenated rosin esters were used instead of alicyclic saturated hydrocarbon resins (Comparative Examples 19 and 20), it was confirmed that the skin permeability of rotigotine decreased.

(Examples 6 and 7)

Each patch was obtained in the same manner as in Example 1 except that the composition of the adhesive agent layer composition was changed to the compositions shown in Table 4 below. The patches obtained in Examples 6 and 7 were subjected to a skin permeation test. Table 4 shows the results together with the compositions (excluding the solvent) of the adhesive agent layer compositions of Examples.

[Table 4]

|  | Example 6 | Example 7 |
|---|---|---|
| Adhesive Agent Layer Composition [Parts by Mass] |  |  |
| Rotigotine | 9 | 9 |
| Styrene-Isoprene-Styrene Block Copolymer | 14.2 | 13.3 |
| Polyisobutylene | 6.0 | 5.7 |
| Alicyclic Saturated Hydrocarbon Resin | 44.6 | 41.8 |
| Liquid Paraffin | 16.2 | 15.2 |
| Octyldodecanol | 10 | 15 |
| Total | 100 | 100 |
| Evaluation |  |  |
| Maximum Rate of Skin Permeation (Jmax) [$\mu$g/cm$^2$/hr] | 17.9 | 17.6 |

As shown in Table 4, it was confirmed that excellent skin permeability of rotigotine was achieved in the patches of the present invention (Examples 6 and 7) even when the content of the aliphatic alcohol according to the present invention was 10 parts by mass or 15 parts by mass.

(Examples 8 to 10)

Each patch was obtained in the same manner as in Example 1 except that the composition of the adhesive agent layer composition was changed to the compositions shown in Table 5 below. The patches obtained in Examples 8 to 10 were subjected to a skin permeation test. Table 5 shows the results together with the compositions (excluding the solvent) of the adhesive agent layer compositions of Examples.

[Table 5]

|  | Example 8 | Example 9 | Example 10 |
|---|---|---|---|
| Adhesive Agent Layer Composition [Parts by Mass] |  |  |  |
| Rotigotine | 9 | 11.3 | 13.5 |
| Styrene-Isoprene-Styrene Block Copolymer | 14.9 | 14.5 | 14.1 |
| Polyisobutylene | 6.3 | 6.1 | 6.0 |
| Alicyclic Saturated Hydrocarbon Resin | 46.8 | 45.5 | 44.3 |
| Liquid Paraffin | 17.0 | 16.6 | 16.1 |
| Octyldodecanol | 5 | 5 | 5 |
| Cross-Linked PVP | 1 | 1 | 1 |
| Total | 100 | 100 | 100 |
| Evaluation |  |  |  |
| Maximum Rate of Skin Permeation (Jmax) [$\mu$g/cm$^2$/hr] | 16.4 | 18.8 | 19.7 |

As shown in Table 5, it was confirmed that excellent skin permeability of rotigotine was achieved in the patches of the present invention (Examples 8 to 10) even when the content of rotigotine was 11.3 parts by mass or 13.5 parts by mass.

(Examples 11 to 15)

Each patch was obtained in the same manner as in Example 1 except that the composition of the adhesive agent layer composition was changed to the compositions shown in Table 6 below. The patches obtained in Examples 11 to 15 were subjected to a skin permeation test. Table 6 shows the results together with the compositions (excluding the solvent) of the adhesive agent layer compositions of Examples. In addition, Table 6 also shows the results of Example 8 above.

[Table 6]

| | Example 11 | Example 12 | Example 13 | Example 8 | Example 14 | Example 15 |
|---|---|---|---|---|---|---|
| Adhesive Agent Layer Composition [Parts by Mass] | | | | | | |
| Rotigotine | 9 | 9 | 9 | 9 | 9 | 9 |
| Styrene-Isoprene-Styrene Block Copolymer | 16.3 | 12.4 | 13.3 | 14.9 | 10.1 | 21.5 |
| Polyisobutylene | 8.7 | 5.3 | 5.7 | 6.4 | 4.3 | - |
| Alicyclic Saturated Hydrocarbon Resin | 12.5 | 39.0 | 41.8 | 46.8 | 60.0 | 47.3 |
| Liquid Paraffin | 47.5 | 28.3 | 15.2 | 17.0 | 11.6 | 17.2 |
| Octyldodecanol | 5 | 5 | 5 | 5 | 5 | 5 |
| Cross-Linked PVP | 1 | 1 | 10 | 1 | - | - |
| Total | 100 | 100 | 100 | 100 | 100 | 100 |
| Evaluation | | | | | | |
| Maximum Rate of Skin Permeation (Jmax) [$\mu$g/cm$^2$/hr] | 18.3 | 16.5 | 16.2 | 16.4 | 14.6 | 19.7 |

As shown in Table 6, it was confirmed that even when the content of alicyclic saturated hydrocarbon resin was 12.5 parts by mass or 60 parts by mass, and even when the content of liquid paraffin was 11.6 parts by mass or 47.5 parts by mass, excellent skin permeability of rotigotine was achieved in the patches of the present invention (Examples 11 to 14). Furthermore, it was confirmed that even without polyisobutylene contained, excellent skin permeability of rotigotine was achieved in the patch of the present invention (Example 15).

[Industrial Applicability]

As described above, it is possible to provide a rotigotine-containing patch with particularly excellent skin permeability of rotigotine.

**Claims**

1. A rotigotine-containing patch comprising:

   a backing layer; and
   an adhesive agent layer, wherein
   the adhesive agent layer contains rotigotine and/or a pharmaceutically acceptable salt thereof, and
   the adhesive agent layer further contains an alicyclic saturated hydrocarbon resin and at least one aliphatic alcohol selected from the group consisting of lauryl alcohol, octyldodecanol, oleyl alcohol, and myristyl alcohol.

2. The rotigotine-containing patch according to claim 1, wherein a content of the aliphatic alcohol in the adhesive agent layer is 1 to 15% by mass relative to a total mass of the adhesive agent layer.

3. The rotigotine-containing patch according to claim 1 or 2, wherein a content of rotigotine and/or a pharmaceutically acceptable salt thereof in the adhesive agent layer in terms of rotigotine free form is 5 to 15% by mass relative to the total mass of the adhesive agent layer.

4. The rotigotine-containing patch according to any one of claims 1 to 3, wherein a content of the alicyclic saturated hydrocarbon resin in the adhesive agent layer is 5 to 80% by mass relative to the total mass of the adhesive agent layer.

5. The rotigotine-containing patch according to any one of claims 1 to 4, wherein the adhesive agent layer further contains a styrene-based thermoplastic elastomer.

6. The rotigotine-containing patch according to claim 5, wherein a content of the styrene-based thermoplastic elastomer in the adhesive agent layer is 5 to 50% by mass relative to the total mass of the adhesive agent layer.

7. The rotigotine-containing patch according to any one of claims 1 to 6, wherein the adhesive agent layer further contains a plasticizer.

8. The rotigotine-containing patch according to claim 7, wherein a content of the plasticizer in the adhesive agent layer is 4 to 50% by mass relative to the total mass of the adhesive agent layer.

**Patentansprüche**

1. Rotigotin enthaltendes Pflaster, das umfasst:

   eine Trägerschicht; sowie
   eine Klebstoffschicht, wobei
   die Klebstoffschicht Rotigotin und/oder ein pharmazeutisch akzeptables Salz desselben enthält und
   die Klebstoffschicht des Weiteren ein alicyclisches gesättigtes Kohlenwasserstoffharz sowie einen aliphatischen Alkohol enthält, der aus der Gruppe ausgewählt wird, die aus Laurylalkohol, Octyldodecanol, Oleylalkohol und Myristylalkohol besteht.

2. Rotigotin enthaltendes Pflaster nach Anspruch 1, wobei ein Gehalt des aliphatischen Alkohols in der Klebstoffschicht, relativ zu einer Gesamtmasse der Klebstoffschicht, 1 bis 15 Masse-% beträgt.

3. Rotigotin enthaltendes Pflaster nach Anspruch 1 oder 2, wobei ein Gehalt an Rotigotin und oder einem pharmazeutisch akzeptablen Salz in der Klebstoffschicht, relativ zu der Gesamtmasse der Klebstoffschicht, bezogen auf Rotigotin in freier Form 5 bis 15 Masse-% beträgt.

4. Rotigotin enthaltendes Pflaster nach einem der Ansprüche 1 bis 3, wobei ein Gehalt an dem alicyclischen gesättigten Kohlenwasserstoffharz in der Klebstoffschicht, relativ zu der Gesamtmasse der Klebstoffschicht, 5 bis 80 Masse-% beträgt.

5. Rotigotin enthaltendes Pflaster nach einem der Ansprüche 1 bis 4, wobei die Klebstoffschicht des Weiteren ein thermoplastisches Elastomer auf Styrol-Basis enthält.

6. Rotigotin enthaltendes Pflaster nach Anspruch 5, wobei ein Gehalt an dem thermoplastischen Elastomer auf Styrol-Basis in der Klebstoffschicht, relativ zu der Gesamtmasse der Klebstoffschicht, 5 bis 50 Masse-% beträgt.

7. Rotigotin enthaltendes Pflaster nach einem der Ansprüche 1 bis 6, wobei die Klebstoffschicht des Weiteren einen Weichmacher enthält.

8. Rotigotin enthaltendes Pflaster nach Anspruch 7, wobei ein Gehalt an dem Weichmacher in der Klebstoffschicht, relativ zu der Gesamtmasse der Klebstoffschicht, 4 bis 50 Masse-% beträgt.

**Revendications**

1. Timbre contenant de la rotigotine comprenant :

   une couche de support ; et
   une couche d'agent adhésif, dans lequel
   la couche d'agent adhésif contient de la rotigotine et/ou un sel pharmaceutiquement acceptable de celle-ci, et

la couche d'agent adhésif contient en outre une résine d'hydrocarbure saturé alicyclique et au moins un alcool aliphatique choisi dans le groupe constitué de l'alcool laurique, de l'octyldodécanol, de l'alcool oléylique et de l'alcool myristylique.

2. Timbre contenant de la rotigotine selon la revendication 1, dans lequel une teneur en alcool aliphatique dans la couche d'agent adhésif est de 1 à 15% en masse par rapport à la masse totale de la couche d'agent adhésif.

3. Timbre contenant de la rotigotine selon la revendication 1 ou 2, dans lequel une teneur en rotigotine et/ou en sel pharmaceutiquement acceptable de celle-ci dans la couche d'agent adhésif en termes de forme libre de rotigotine est de 5 à 15% en masse par rapport à la masse totale de la couche d'agent adhésif.

4. Timbre contenant de la rotigotine selon l'une quelconque des revendications 1 à 3, dans lequel une teneur en résine d'hydrocarbure saturé alicyclique dans la couche d'agent adhésif est de 5 à 80% en masse par rapport à la masse totale de la couche d'agent adhésif.

5. Timbre contenant de la rotigotine selon l'une quelconque des revendications 1 à 4, dans lequel la couche d'agent adhésif contient en outre un élastomère thermoplastique à base de styrène.

6. Timbre contenant de la rotigotine selon la revendication 5, dans lequel une teneur en élastomère thermoplastique à base de styrène dans la couche d'agent adhésif est de 5 à 50% en masse par rapport à la masse totale de la couche d'agent adhésif.

7. Timbre contenant de la rotigotine selon l'une quelconque des revendications 1 à 6, dans lequel la couche d'agent adhésif contient en outre un plastifiant.

8. Timbre contenant de la rotigotine selon la revendication 7, dans lequel une teneur en plastifiant dans la couche d'agent adhésif est de 4 à 50% en masse par rapport à la masse totale de la couche d'agent adhésif.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010159302 A
- JP 2002509878 A
- WO 2012084969 A
- JP 2014177428 A
- JP 2018012331 A
- JP 2013079220 B
- JP 2018123131 A

**Non-patent literature cited in the description**

- Japanese Pharmaceutical Excipients Directory. International Pharmaceutical Excipients Council Japan, 2016